# EUROPÄISCHE PATENTANMELDUNG

(11) **EP 4 635 439 A1**
(43) Veröffentlichungstag der Anmeldung: **22.10.2025**
(21) Anmeldenummer: 25171406.9
(22) Anmeldetag: 17.04.2025
(51) Int. Cl.: A61B 18/12, A61B 18/04, H05H 1/24, A61B 18/00

(54) **ENERGIEVERSORGUNGSVORRICHTUNG**

(30) Priorität: 18.04.2024 DE 102024110877
(71) Anmelder: Karl Storz SE & Co. KG, 78532 Tuttlingen (DE)
(72) Erfinder: HINDING, Thomas, 78532 Tuttlingen (DE); JÉRÔME, Pierre, 78532 Tuttlingen (DE)

(57) **Zusammenfassung**

Energieversorgungsvorrichtung für ein elektrochirurgischen Instruments. Hierbei sind mehrere Betriebsphasen vorgesehen. In einer ersten Betriebsphase zum Zünden eines Plasmas an dem elektrochirurgischen Instrument werden mehrere PWM-Stufen zeitsynchron angesteuert, um einen rechteckförmigen Spannungsverlauf zu generieren. In einer zweiten Betriebsphase zum Aufrechterhalten des Plasmas werden die mehreren PWM-Stufen individuell und mit gegebenenfalls mehreren Schaltvorgängen pro Periodendauer des zu generierenden Ausgangssignals angesteuert.

## Beschreibung

### Technisches Gebiet der Erfindung

Die vorliegende Erfindung betrifft eine Energieversorgungsvorrichtung für ein elektrochirurgisches Instrument. Die vorliegende Erfindung betrifft ferner ein elektrochirurgisches System mit einer solchen Energieversorgungsvorrichtung sowie ein Verfahren zum Bereitstellen einer hochfrequenten Versorgungsspannung für ein elektrochirurgisches Instrument.

### Hintergrund der Erfindung

Bei medizinischen Eingriffen mittels minimalinvasiver Endoskopie ist eine schnelle und zuverlässige Gewebeversiegelung von großer Bedeutung, da bereits eine sehr geringe Mengen von Blut die Sicht eines Operateurs behindern kann. Dies könnte den durchgeführten Eingriff erschweren oder gegebenenfalls gar unmöglich machen. Zur Versiegelung des Gewebes kann beispielsweise mittels eines entsprechenden elektrochirurgischen Instruments mit einem hochfrequenten Strom das relevante Gewebe erhitzt und hierdurch versiegelt werden. Auf diese Weise kann eine mögliche Blutung sehr rasch gestoppt werden.

Für derartige Anwendungen können beispielsweise elektrochirurgische Systeme zum Einsatz kommen. Ein elektrochirurgisches System kann beispielsweise einen HochfrequenzGenerator umfassen, welcher ein Hochfrequenzsignal erzeugt, das an einer Hochfrequenzelektrode angelegt werden kann. Während einer Hochspannungsentladung an dieser Elektrode bildet sich ein Plasma, welches für eine medizinische Behandlung wie zum Beispiel zur Stillung einer Blutung (Koagulation) genutzt werden kann.

Der Betrieb eines solchen elektrochirurgischen Systems kann in zwei Phasen unterteilt werden. In einer ersten Betriebsphase (Zündphase) ist es zunächst erforderlich, das Plasma an der Hochfrequenzelektrode zu zünden. Hierbei ist eine relativ hohe elektrische Leistung erforderlich. In der sich daraufhin anschließenden weiteren Phase muss das gezündete Plasma aufrecht erhalten werden. In dieser weiteren Phase ist eine gegenüber der Zündphase deutlich geringere Leistung ausreichend und gewünscht, um beispielsweise den Energieeintrag an der Behandlungsstelle möglichst gering zu halten. Dabei ist es jedoch erforderlich, die elektrische Leistung während der Behandlung möglichst präzise zu steuern.

Vor diesem Hintergrund ist eine effiziente, sichere und kostengünstige Energieversorgung für ein solches elektrochirurgisches System wünschenswert. Insbesondere ist eine Vorrichtung wünschenswert, welche die erforderliche hochfrequente Spannung zum Zünden und Betreiben eines elektrochirurgischen Instruments auf möglichst einfache Weise und mit der erforderlichen Präzision bereitstellen kann.

### Zusammenfassung der Erfindung

Die vorliegende Erfindung schafft eine Energieversorgungsvorrichtung für ein elektrochirurgisches Instrument, ein elektrochirurgisches System und ein Verfahren zum Bereitstellen einer hochfrequenten Versorgungsspannung für ein elektrochirurgisches Instrument mit den Merkmalen der unabhängigen Patentansprüche. Weitere vorteilhafte Ausführungsformen sind Gegenstand der abhängigen Patentansprüche.

Gemäß einem ersten Aspekt schafft die vorliegende Erfindung eine Energieversorgungsvorrichtung für ein elektrochirurgisches Instrument. Die Energieversorgungsvorrichtung umfasst mehrere PWM-Stufen, einen Transformator sowie eine Steuereinrichtung.

Jede PWM-Stufe umfasst einen Eingangsanschluss und einen Ausgangsanschluss. Die PWM-Stufen sind dazu ausgelegt, an ihrem Eingangsanschluss mit einer Gleichspannungsquelle elektrisch gekoppelt zu werden. Die Ausgangsanschlüsse der mehreren PWM-Stufen sind an einem gemeinsamen Knotenpunkt elektrisch miteinander gekoppelt. Mit anderen Worten, die mehreren PWM-Stufen speisen ihre Ausgangssignale auf diesen gemeinsamen Knotenpunkt. Der Transformator umfasst eine Primärseite und eine Sekundärseite. Ein erster Anschlusspunkt der Primärseite des Transformators ist mit dem gemeinsamen Knotenpunkt der mehreren PWM-Stufen elektrisch gekoppelt. Der zweite Anschlusspunkt der Primärseite des Transformators kann mit einem Bezugspotential verbunden sein.

Der Transformator ist ferner dazu ausgelegt, zwischen einem ersten Anschlusspunkt und einem zweiten Anschlusspunkt der Sekundärseite eine Versorgungsspannung für das elektrochirurgische Instrument bereitzustellen. Auf diese Weise kann eine von der Gleichspannungsquelle galvanisch getrennte elektrische Ausgangsspannung zur Versorgung des elektrochirurgischen Instruments bereitgestellt werden.

Die Steuereinrichtung ist dazu ausgelegt, Ansteuersignale für die mehreren PWM-Stufen bereitzustellen. Insbesondere kann die Steuereinrichtung zwischen mindestens zwei Betriebsmodi unterscheiden. In einem ersten Betriebsmodus kann die Steuereinrichtung die mehreren PWM-Stufen zeitsynchron anzusteuern. Mit anderen Worten stellt die Steuereinrichtung an allen PWM-Stufen gleiche Ansteuersignal bereit. In einem zweiten Betriebsmodus kann die Steuereinrichtung die mehreren PWM-Stufen zu unterschiedlichen Zeitpunkten anzusteuern. Mit anderen Worten stellt die Steuereinrichtung an den verschiedenen PWM-Stufen jeweils individuelle Steuersignale bereit, wobei die Flanken für das Ein- bzw. Ausschalten in den Steuersignalen für die einzelnen PWM-Stufen zu unterschiedlichen Zeitpunkten vorgesehen sein können.

Gemäß einem weiteren Aspekt ist ein elektrochirurgisches System vorgesehen. Das elektrochirurgische System umfasst ein elektrochirurgisches Instrument sowie eine erfindungsgemäße Energieversorgungsvorrichtung. Das elektrochirurgische Instrument umfasst mindestens eine Elektrode, an der ein Plasma erzeugt werden kann.

Gemäß einem weiteren Aspekt ist ein Verfahren zum Bereitstellen einer hochfrequenten Versorgungsspannung für ein elektrochirurgisches Instrument vorgesehen. Das Bereitstellen der hochfrequenten Versorgungsspannung kann insbesondere mittels einer erfindungsgemäßen Energieversorgungsvorrichtung, insbesondere einer Energieversorgungsvorrichtung gemäß dem ersten Aspekt erfolgen. Das Verfahren umfasst einen Schritt zum zeitsynchronen Ansteuern der mehreren PWM-Stufen in einer ersten Betriebsphase. Diese erste Betriebsphase kann zum Zünden eines Plasmas an dem elektrochirurgischen Instrument vorgesehen sein. Weiterhin umfasst das Verfahren einen Schritt zum zeitlich versetzten Ansteuern der mehreren PWM-Stufen in einer zweiten Betriebsphase. Diese zweite Betriebsphase kann zum Aufrechterhalten des Plasmas an dem elektrochirurgischen Instrument vorgesehen sein. Insbesondere kann sich die zweite Betriebsphase der ersten Betriebsphase unmittelbar anschließen.

Der vorliegenden Erfindung liegt die Erkenntnis zugrunde, dass sich der Betrieb eines elektrochirurgischen Instruments in der Regel in mindestens zwei Phasen gliedert. In einer ersten Betriebsphase, während der zunächst ein Plasma an einer Elektrode des elektrochirurgischen Instruments gezündet werden soll, muss dabei eine relativ hohe elektrische Leistung bereitgestellt werden. In einer sich daraufhin anschließenden zweiten Betriebsphase, in der dieses Plasma aufrecht erhalten werden soll, ist eine geringere elektrische Leistung erforderlich. Hierbei ist jedoch eine möglichst präzise Steuerung der elektrischen Leistung in der zweiten Betriebsphase während des Aufrechterhaltens wünschenswert. Während der ersten Betriebsphase (Zündphase) sind die Anforderungen an die Regelung elektrischen Leistung dagegen geringer als in der zweiten Betriebsphase.

Ausgehend von dieser Erkenntnis ist es daher eine Idee der vorliegenden Erfindung, eine Energieversorgung für ein solches elektrochirurgisches Instrument zu schaffen, welches einerseits die für das Zünden des Plasmas erforderliche elektrische Leistung bereitstellen kann und darüber hinaus andererseits auch die für das während des Aufrechterhaltens des Plasmas gewünschte Regelgenauigkeit gewährleisten kann. Insbesondere können diese Anforderungen gemäß dem erfindungsgemäßen Ansatz durch einen besonders kompakten, effizienten und somit auch kostengünstigen Aufbau realisiert werden.

Hierzu ist es vorgesehen, die elektrische Leistung für den Betrieb des elektrochirurgischen Instruments sowohl während der ersten Betriebsphase zum Zünden des Plasmas als auch während der zweiten Betriebsphase zum Aufrechterhalten des Plasmas mittels derselben elektrischen Schaltung bereitzustellen. Insbesondere ist hierzu ein Schaltungskonzept mit mehreren parallelen PWM-Stufen vorgesehen. Jede dieser PWM-Stufen kann mittels eines Ansteuersignals, insbesondere eines pulsbreitenmodulierten (Pulse Width Modulation - PWM) Ansteuersignals, angesteuert werden.

Während einer ersten Betriebsphase zum Zünden des Plasmas können dabei alle PWM-Stufen auf Grundlage eines gemeinsamen PWM-Signals angesteuert werden. Hierdurch werden alle PWM-Stufen gemeinsam geschaltet. Dies führt zu einer - im Vergleich zu der nachfolgend beschriebenen zweiten Betriebsphase - relativ geringen Anzahl von Schaltvorgängen. Hierbei wird als Ausgangssignal ein rechteckförmiges Spannungssignal generiert. Eine solche Ansteuerung kann ein Ausgangssignal mit einer relativ hohen Effizienz erzeugen. Diese gute Effizienz ermöglicht das Bereitstellen einer relativ hohen Ausgangsleistung mit vergleichbar geringem Schaltungsaufwand bzw. geringer dimensionierten Schaltungskomponenten.

In der sich anschließenden zweiten Betriebsphase zum Aufrechterhalten des Plasmas werden die einzelnen PWM-Stufen jeweils mit individuellen PWM-Signalen angesteuert, wobei sich diese individuellen PWM-Signale in den Schaltzeitpunkten für die jeweiligen PWM-Stufen unterschreiten können. Auch können in den einzelnen Ansteuersignalen für die PWM-Stufen pro Periodendauer des hochfrequenten Ausgangssignals mehrere Ein- und Ausschaltvorgänge vorgesehen sein. Auf diese Weise ist es möglich, ein Ausgangssignal zu generieren, dessen Signalform in sehr guter Näherung eine Sinusform erreichen kann. Derartige Spannungsformen sind im Vergleich zu den rechteckförmigen Spannungsformen während der Zündphase besser mess- und regelbar. Auf diese Weise kann die elektrische Leistung während dieser Betriebsphase sehr gut überwacht und geregelt werden.

Durch diesen Ansatz, bei welchem dieselben Schaltungsbaugruppen, insbesondere dieselben PWM-Stufen, in den beiden Betriebsphasen auf unterschiedliche Weise angesteuert werden, ist es möglich, einerseits während des Zündens des Plasmas die Schaltung in einem Modus mit einer hohen Effizienz zu betreiben und so die für das Zünden des Plasmas erforderliche hohe elektrische Leistung bereitstellen zu können.

Darüber hinaus kann durch den Wechsel der Ansteuerung in der zweiten Betriebsphase für das Aufrechterhalten des Plasmas eine sehr gute Regelung der elektrischen Leistung erzielt werden. Da während dieser zweiten Betriebsphase der Leistungsbedarf während des Aufrechterhaltens des Plasmas geringer ist als der Leistungsbedarf für das Zünden des Plasmas, kann die geringere Effizienz bei der Ansteuerung in der zweiten Betriebsphase in Kauf genommen werden. Folglich kann die Energieversorgung für den Betrieb eines elektrochirurgischen Instruments mit einer einzigen Schaltungsanordnung realisiert werden, wobei diese Schaltungsanordnung durch den sehr effizienten Betrieb während des Zündens des Plasmas deutlich geringer und somit auch kleiner und kostengünstiger realisiert werden kann, als dies der Fall wäre, wenn nur eine einzige Ansteuerung entsprechend dem zweiten Betriebsmodus vorgesehen würde.

Gemäß einer Ausführungsform ist die Steuereinrichtung dazu ausgelegt, die mehreren PWM-Stufen in dem zweiten Betriebsmodus mit einer Taktrate anzusteuern, welche größer ist als die Taktrate in dem ersten Betriebsmodus. Insbesondere können in dem zweiten Betriebsmodus pro Periodendauer des hochfrequenten Ausgangssignals in den Ansteuersignalen mehrere Ein- und Ausschaltvorgänge vorgesehen sein. Hierdurch kann die Signalform der Ausgangsspannung sehr gut einer gewünschten Sinusform angenähert werden

Gemäß einer Ausführungsform umfasst die Ansteuerung in dem zweiten Betriebsmodus eine Multiphasen-PWM-Ansteuerung. Bei einer solchen Multiphasen-PWM-Ansteuerung werden die einzelnen PWM-Stufen jeweils mit einer gemeinsamen Taktfrequenz angesteuert. Die Einschalt- und Ausschaltzeitpunkte der einzelnen PWM-Stufen können sich hierbei jedoch voneinander unterscheiden. Auch können gegebenenfalls die Tastverhältnisse für die Ansteuersignal der einzelnen PWM-Stufen unterschiedlich sein. Durch eine solche Multiphasen-PWM-Ansteuerung kann als gemeinsames Ausgangssignal der PWM-Stufen ein sinusförmiger Spannungsverlauf sehr gut angenähert werden.

Gemäß einer Ausführungsform ist eine Amplitude der Versorgungsspannung für das elektrochirurgische Instrument , die zwischen dem ersten Anschlusspunkt und einem zweiten Anschlusspunkt der Sekundärseite des Transformators bereitgestellt wird, größer als eine elektrische Eingangsspannung der Gleichspannungsquelle, die an den Eingangsanschlüssen der PWM-Stufen bereitgestellt wird und die PWM-Stufen mit elektrischer Energie versorgt. Mit anderen Worten, die erfindungsgemäße Schaltungsanordnung zur Energieversorgung des elektrochirurgischen Instruments gibt eine elektrische Spannung aus, welche (signifikant) größer ist als die Eingangsspannung dieser Schaltungsanordnung. Somit ist es möglich, die eingangsseitigen Bauelemente, insbesondere die PWM-Stufen, auf eine relativ niedrige elektrische Spannung zu dimensionieren. Hierdurch können Bauelemente gewählt werden, welche ein geringes Bauraumvolumen aufweisen und welche auch kostengünstig verfügbar sind. Für die Anhebung der elektrischen Spannung auf das für den Betrieb des elektrochirurgischen Instruments erforderliche Spannungshöhe kann daraufhin unter anderem der nachgeschaltete Transformator vorgesehen sein.

Gemäß einer Ausführungsform ist die Eingangsspannung der Gleichspannungsquelle kleiner oder gleich 60 Volt. Insbesondere kann die Eingangsspannung der Gleichspannungsquelle, welche an den Eingängen der PWM-Stufen bereitgestellt wird, sich im Bereich der Schutzkleinspannung befinden. Auf diese Weise kann die Energieversorgungsvorrichtung für das elektrochirurgische Instrument mit einer sicheren Eingangsspannung betrieben werden. Hierdurch kann eine mögliche Gefahr, beispielsweise für einen Benutzer, durch einen elektrischen Schlag o. ä. vermieden werden. Das Bereitstellen einer solchen elektrischen Gleichspannung kann beispielsweise mittels einer separaten, externen Komponente, wie zum Beispiel einem entsprechenden Netzgerät oder ähnlichem bereitgestellt werden. Insbesondere kann hierbei ein galvanisch trennendes Netzgerät vorgesehen sein, wodurch die Sicherheit weiter erhöht werden kann.

Gemäß einer Ausführungsform umfasst die Energieversorgungsvorrichtung einen Koppelkondensator. Der Koppelkondensator kann zwischen dem gemeinsamen Knotenpunkt der mehreren PWM-Stufen und dem ersten Anschlusspunkt der Primärseite des Transformators angeordnet sein. Insbesondere kann der Koppelkondensator für (oder: an) die zu übertragende elektrische Leistung und/oder die Frequenz des übertragenen elektrischen Signals angepasst sein. Durch einen solchen Koppelkondensator können beispielsweise Gleichspannungsanteile bzw. ein Gleichspannungs-Offset für die an dem Transformator anliegende elektrische Wechselspannung ausgekoppelt werden.

Gemäß einer Ausführungsform ist die Steuereinrichtung dazu ausgelegt, einen elektrischen Eingangsstrom von der Gleichspannungsquelle zu den Eingangsanschlüssen der PWM-Stufen zu ermitteln. Hierzu können beliebige geeignete Verfahren sowie Komponenten vorgesehen sein. Beispielsweise kann der elektrische Eingangsstrom mittels eines oder mehrerer Stromsensoren ermittelt werden. Bei einer solchen Konfiguration kann die Steuereinrichtung die mehreren PWM-Stufen unter Verwendung des ermittelten Eingangsstroms ansteuern. Beispielsweise kann bei einer bekannten oder ebenfalls ermittelten Eingangsspannung aus dem Eingangsstrom die elektrische Leistung bestimmt werden. Somit kann auf Grundlage des ermittelten Eingangsstrom eine Steuerung bzw. Regelung der elektrischen Leistung für das elektrochirurgische Instrument realisiert werden.

Ferner ist es beispielsweise auch möglich, unter Verwendung des ermittelten Eingangsstroms das Zünden des Plasmas zu detektieren. Somit kann nach dem Zünden des Plasmas von dem ersten Betriebsmodus in den zweiten Betriebsmodus gewechselt werden. Ferner ist es beispielsweise möglich, aus dem elektrischen Eingangsstrom auch ein (unbeabsichtigtes) Erlöschen des Plasmas zu detektieren. In einem solchen Fall kann beispielsweise (automatisch) zurück in die erste Betriebsphase gewechselt werden, um das Plasma erneut zu zünden. Selbstverständlich kann der ermittelte elektrische Eingangsstrom auch für beliebige weitere Anwendungen genutzt werden.

Gemäß einer Ausführungsform ist die Steuereinrichtung dazu ausgelegt, die mehreren PWM-Stufen in dem ersten Betriebsmodus anzusteuern, falls an einem an dem Ausgangsanschluss angeschlossenen elektrochirurgischen Instrument kein Plasma detektiert wird. Beispielsweise kann in einer Startphase zunächst davon ausgegangen werden, dass noch kein Plasma existiert und daher das Plasma gezündet werden soll. Gegebenenfalls kann das Zünden des Plasmas auch durch eine manuelle Eingabe, beispielsweise durch einen Benutzer, getriggert werden. Ferner sind jedoch auch beliebige geeignete Detektionsverfahren, wie beispielsweise das Überwachen eines elektrischen Stroms oder einer elektrischen Leistung möglich, um festzustellen, ob ein Plasma vorhanden ist oder nicht. Liegt kein Plasma vor, so kann gegebenenfalls automatisch in den ersten Betriebsmodus gewechselt werden, um das Plasma zu zünden.

Gemäß einer Ausführungsform ist die Steuereinrichtung dazu ausgelegt, die mehreren PWM-Stufen in dem zweiten Betriebsmodus anzusteuern, falls an einem an dem Ausgangsanschluss angeschlossenen elektrochirurgischen Instrument ein Plasma detektiert wird. Hierzu sind beliebige geeignete Verfahren, wie beispielsweise eine Überwachung eines elektrischen Stroms oder einer elektrischen Leistung möglich, um ein stabiles Plasma an dem elektrochirurgischen Instrument zu erkennen.

Gemäß einer Ausführungsform ist die Steuereinrichtung dazu ausgelegt, nach einer vorbestimmten Zeitspanne von dem ersten Betriebsmodus in den zweiten Betriebsmodus zu wechseln. Beispielsweise kann zunächst für eine fest vorbestimmte Zeitspanne die Energieversorgung in dem ersten Betriebsmodus erfolgen, um ein Zünden des Plasmas zu ermöglichen. Nach Ablauf dieser Zeitspanne kann daraufhin gegebenenfalls automatisch in den zweiten Betriebsmodus gewechselt werden, um ein gezündetes Plasma aufrechtzuerhalten. Die vorbestimmte Zeitspanne, nach der von dem ersten Betriebsmodus in den zweiten Betriebsmodus gewechselt wird, kann beispielsweise maximal 500 ms, beispielsweise 200 ms oder aber auch eine hiervon abweichende Zeitspanne von zum Beispiel 100 ms, 300 ms, 400 ms oder eine beliebige andere geeignete Zeitspanne umfassen.

Gemäß einer Ausführungsform umfasst die Steuereinrichtung einen FPGA (Field Programmable Gate Array). Ein solcher FPGA kann dazu ausgelegt sein, die Ansteuersignale für die mehreren PWM-Stufen zu erzeugen und bereitzustellen. Beispielsweise kann jeweils ein Ausgangsanschluss des FPGA ein Ansteuersignal für eine PWM-Stufe bereitstellen. Grundsätzlich sind jedoch je nach Anwendungsfall auch beliebige andere geeignete Schaltungskonzepte sowie andere geeignete Bauelemente bzw. Schaltkreise zur Erzeugung der Ansteuersignale für die PWM-Stufen möglich.

Gemäß einer Ausführungsform umfassen die mehreren PWM-Stufen jeweils eine Halbbrücke. Jede Halbbrücke kann hierbei zwei Halbleiterschaltelemente umfassen. Beispielsweise kann ein erstes Halbleiterschaltelement zwischen einem Eingangsanschluss einer PWM-Stufe und einem Ausgangsanschluss der PWM-Stufe vorgesehen sein, und ein zweites Halbleiterschaltelement kann zwischen dem Ausgangsanschluss der PWM-Stufe und einem Bezugspotenzial vorgesehen sein. Alternativ kann ein erstes Halbleiterschaltelement beispielsweise zwischen einem positiven Anschluss der Gleichspannungsquelle und dem Ausgangsanschluss vorgesehen sein, und ein zweites Halbleiterschaltelement kann zwischen dem Ausgangsanschluss und einem negativen Anschluss der Gleichspannungsquelle vorgesehen sein. Je nach Anwendungsfall sind jedoch auch hiervon abweichende Schaltungskonzepte für die einzelnen PWM-Stufen möglich.

Gemäß einer Ausführungsform ist die Steuereinrichtung dazu ausgelegt, die mehreren PWM-Stufen jeweils mit einer Taktfrequenz von mindestens 300 kHz anzusteuern. Derart hohe Frequenzen führen in der Regel zu keiner Reizung der Nerven bei dem Patienten und sind daher besonders gut geeignet. Grundsätzlich sind jedoch auch andere Frequenzen, insbesondere Frequenzen von mehr als 300 kHz, beispielsweise 500 kHz, 800 kHz, 1 MHz, 2 MHz o. ä. möglich.

Die obigen Ausgestaltungen und Weiterbildungen lassen sich, soweit sinnvoll, beliebig miteinander kombinieren. Weitere Ausgestaltungen, Weiterbildungen und Implementierungen der Erfindung umfassen auch nicht explizit genannte Kombinationen von zuvor oder im Folgenden bezüglich den Ausführungsbeispielen beschriebenen Merkmalen der Erfindung. Insbesondere wird der Fachmann auch Einzelaspekte als Verbesserungen oder Ergänzungen zu den jeweiligen Grundformen der Erfindung hinzufügen.

### Kurze Beschreibung der Zeichnungen

Weitere Merkmale und Vorteile der Erfindung werden nachfolgend anhand der Figuren erläutert. Dabei zeigen:
- Fig. 1:: eine schematische Darstellung eines elektrochirurgischen Systems gemäß einer Ausführungsform;
- Fig. 2:: eine schematische Darstellung eines Prinzipschaltbilds für eine Energieversorgungsvorrichtung eines elektrochirurgischen Systems gemäß einer Ausführungsform;
- Fig. 3:: ein Timing-Diagramm zur Veranschaulichung der Ansteuersignale in einem ersten Betriebsmodus der Energieversorgungsvorrichtung gemäß einer Ausführungsform;
- Fig. 4:: ein Timing-Diagramm zur Veranschaulichung der Ansteuersignal in einem zweiten Betriebsmodus der Energieversorgungsvorrichtung gemäß einer Ausführungsform; und
- Fig. 5:: ein Ablaufdiagramm, wie es einem Verfahren zum Bereitstellen einer hochfrequenten Versorgungsspannung für ein elektrochirurgisches Instrument gemäß einer Ausführungsform zugrunde liegen kann.

### Beschreibung von Ausführungsformen

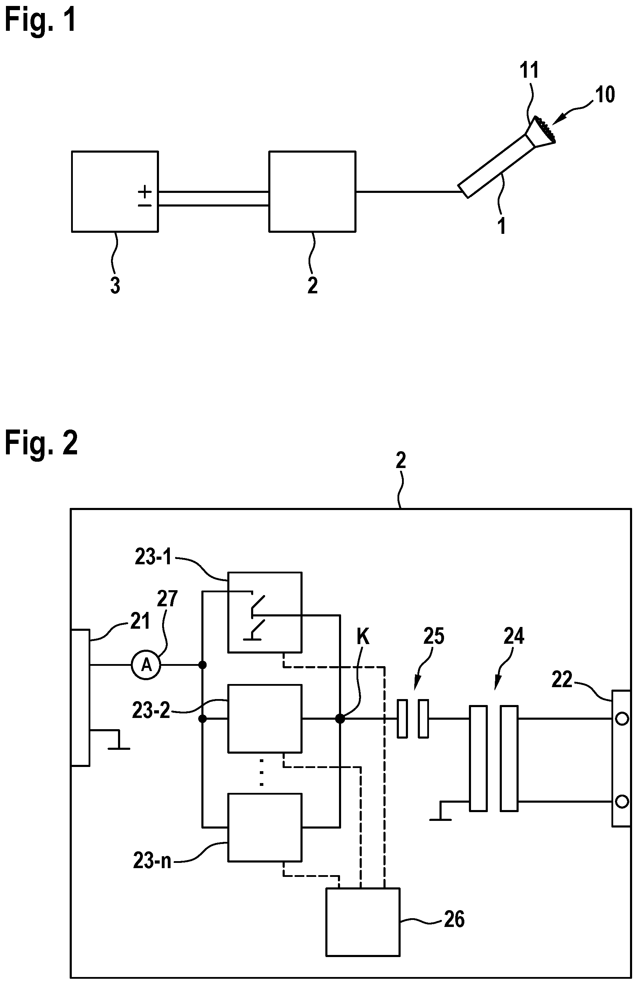

Figur 1 zeigt eine schematische Darstellung eines Blockschaubilds eines elektrochirurgischen Systems gemäß einer Ausführungsform. Das elektrochirurgische System umfasst ein elektrochirurgisches Instrument 1, insbesondere ein elektrochirurgisches Instrument 1 zu Erzeugung eines Plasmas 10. Das Plasma 10 kann hierbei an einer Elektrode 11 bzw. zwischen zwei Elektroden 11 erzeugt werden. Durch ein solches Plasma 10 kann zum Beispiel Gewebe in einem Patienten versiegelt werden, um beispielsweise eine Blutung zu stoppen. Grundsätzlich sind jedoch auch beliebige andere geeignete Anwendungen möglich. Insbesondere kann es sich bei dem elektrochirurgischen Instrument 1 um ein medizinisches Instrument für eine minimalinvasive Endoskopie handeln.

Für den Betrieb des elektrochirurgischen Instruments 1 und insbesondere zur Erzeugung des Plasmas 10 ist elektrische Energie erforderlich. Diese elektrische Energie kann von einer Energieversorgungsvorrichtung 2 bereitgestellt werden. Bei der von der Energieversorgungseinrichtung 2 bereitgestellten elektrischen Energie kann es sich insbesondere um eine hochfrequente elektrische Spannung handeln. Insbesondere kann die hochfrequente elektrische Spannung eine Frequenz von mindestens 300 kHz aufweisen. Zum Beispiel kann die hochfrequente elektrische Spannung eine Frequenz von 300 kHz, 400 kHz, 500 kHz, 700 kHz, 1 MHz oder 2 MHz aufweisen. Gegebenenfalls kann die Frequenz auch innerhalb eines vorgebbaren Frequenzbereichs variiert oder eingestellt werden. Beispielsweise kann die Frequenz in einem Bereich zwischen 300 kHz und 600 kHz eingestellt werden. Je nach Anwendungsfall sind jedoch auch andere Frequenzen bzw. Frequenzbereiche möglich.

Die Spannungshöhe der hochfrequenten elektrischen Spannung kann auf einen beliebigen geeigneten Wert eingestellt werden, der zum Zünden bzw. Aufrechterhalten des Plasmas 10 geeignet ist. Beispielsweise kann die elektrische Spannung einen Effektivwert von 100 V oder mehr aufweisen. Je nach Anwendungsfall sowie Konfiguration des elektrochirurgischen Instruments 1 können jedoch auch höhere elektrische Spannungen von 200 V, 500 V, 1 kV oder mehr vorgesehen seien. Insbesondere kann während des Betriebs des elektrochirurgischen Instruments 1 die in dem Plasma 10 umgesetzte elektrische Leistung durch eine geeignete Steuerung eingestellt oder begrenzt werden. Hierzu kann beispielsweise auch eine Regelung oder Steuerung auf Grundlage eines elektrischen Stromes vorgesehen sein. In diesem Fall kann die elektrische Spannung auch derart eingestellt werden, dass der elektrische Strom oder die in dem Plasma umgesetzt elektrische Leistung auf einen vorgegebenen Wert eingestellt oder begrenzt wird. Je nach Anwendungsfall sind darüber hinaus auch andere geeignete Parameter zur Steuerung bzw. Regelung möglich.

Zur Energieversorgung des elektrochirurgischen Instruments 1 kann die Energieversorgungsvorrichtung 2 die hochfrequente elektrische Spannung zum Betrieb des elektrochirurgischen Instruments 1 bereitstellen. Beispielsweise kann die Energieversorgungsvorrichtung 2 die erforderliche hochfrequente elektrische Spannung aus einer an der Energieversorgungsvorrichtung 2 eingangsseitig bereitgestellten Gleichspannung erzeugen. Diese Eingangs-Gleichspannung kann zum Beispiel von einer Gleichspannungsquelle 3 bereitgestellt werden. Bei dieser Gleichspannungsquelle 3 kann es sich zum Beispiel um ein Netzgerät handeln, welches aus einer elektrischen Wechselspannung eines Niederspannungs-Energieversorgungsnetzes mit einer Spannungshöhe von beispielsweise 230 V AC eine geeignete Gleichspannung erzeugt und bereitgestellt. Insbesondere kann eine galvanische Trennung zwischen dem Eingang und dem Ausgang des Netzgeräts vorgesehen sein. Somit kann auch bei einem möglichen Fehlerfall, wie zum Beispiel einem Isolationsfehler, eine zusätzliche Sicherheit des Benutzers vor einem elektrischen Schlag gewährleistet werden.

Die von der Gleichspannungsquelle 3 bereitgestellte elektrische Gleichspannung kann als Eingangsspannung der Energieversorgungsvorrichtung 2 bereitgestellt werden. Insbesondere kann diese Eingangsspannung für die Energieversorgungsvorrichtung 2 eine Schutzkleinspannung, wie zum Beispiel eine elektrische Gleichspannung von maximal 60 V aufweisen. Zum Beispiel kann die Eingangsspannung eine Spannungshöhe von 48 V, 24 V, 12 V oder 5 V aufweisen. Selbstverständlich sind auch beliebige andere geeignete Spannungswerte möglich. Die Eingangsgleichspannung für die Energieversorgungsvorrichtung 2 kann bevorzugt eine Spannungshöhe aufweisen, welche kleiner ist als eine Amplitude der hochfrequenten Ausgangsspannung zur Energieversorgung des elektrochirurgischen Instruments 1. Mit anderen Worten, die Energieversorgungsvorrichtung 2 konvertiert nicht nur die Eingangsgleichspannung in eine hochfrequente Wechselspannung, sondern erhöht darüber hinaus auch noch das Spannungsniveau. Aufgrund der Eigenschaft, dass die Eingangsgleichspannung kleiner ist als die gewünschte Ausgangsspannung, können die eingangsseitigen Bauelemente, insbesondere die Bauelemente zur Erzeugung der hochfrequenten Spannung sowie zur Strom- und/oder Spannungsregelung auf das niedrige eingangsseitige Spannungsniveau ausgelegt sein.

Figur 2 zeigt eine schematische Darstellung eines Prinzipschaltbilds für eine Energieversorgungsvorrichtung 2 gemäß einer Ausführungsform. Die Energieversorgungsvorrichtung 2 kann beispielsweise einen Eingangsanschluss 21 aufweisen. An diesem Eingangsanschluss 21 kann zum Beispiel eine eingangsseitige Gleichspannung, wie die Gleichspannung von der Gleichspannungsquelle 3 bereitgestellt werden. Ferner kann die Energieversorgungsvorrichtung 2 einen Ausgangsanschluss 22 aufweisen. An diesem Ausgangsanschluss 22 kann zum Beispiel das elektrochirurgische Instrument 1 angeschlossen werden.

Die Energieversorgungsvorrichtung 2 kann mehrere PWM-Stufen 23-i umfassen. Die PWM-Stufen 23-i sind eingangsseitig jeweils mit dem Eingangsanschluss 21 der Energieversorgungsvorrichtung 2 verbunden. Die Ausgänge der PWM-Stufen 23-i sind jeweils mit einem gemeinsamen Knotenpunkt K verbunden. Die PWM-Stufen 23-i können mittels geeigneter Steuersignale von einer Steuereinrichtung 26 angesteuert werden. Hierzu kann die Steuereinrichtung 26 für jede PWM-Stufe 23-i ein pulsbreitenmoduliertes (pulse width modulated - PWM) Taktsignal erzeugen und an der jeweiligen PWM-Stufe 23-i bereitstellen. Für die Berechnung sowie Erzeugung dieser PWM-Signale kann in der Steuereinrichtung 26 zum Beispiel ein FPGA vorgesehen sein. Grundsätzlich kann die Erzeugung der PWM-Signale jedoch auch auf beliebige andere geeignete Weise realisiert werden. Die Erzeugung bzw. die Eigenschaften der PWM-Signale wird nachfolgend noch näher erläutert.

In den einzelnen PWM-Stufen 23-i können beliebige geeignete Schaltungsstrukturen vorgesehen sein, wodurch in Abhängigkeit des Zustandes des jeweils anliegenden Steuersignals von der Steuereinrichtung 26 Schaltelemente geöffnet bzw. geschlossen werden. Zum Beispiel kann in jeder PWM-Stufe 23-i eine Halbbrücke aus einer Serienschaltung von zwei Halbleiterschaltelementen vorgesehen sein. Beispielsweise kann ein erstes Halbleiterschaltelement M1 zwischen einem positiven Anschlusspunkt des Eingangsanschlusses 21 und einem Ausgangsanschluss der jeweiligen PWM-Stufe 23-i vorgesehen sein, und ein zweites Halbleiterschaltelement M2 kann zwischen dem Ausgangsanschluss der PWM-Stufe 23-i und einem Bezugspotenzial bzw. einem negativen Anschlusspunkt des Eingangsanschlusses 22 vorgesehen sein. Bei einer solchen Konfiguration kann zum Beispiel bei einer logischen 1 des Taktsignals das erste Halbleiterschaltelement M1 geschlossen werden und das zweite Halbleiterschaltelement M2 geöffnet werden, so dass der Ausgangsanschluss der jeweiligen PWM-Stufe 23-i mit dem positiven Anschlusspunkt des Eingangsanschlusses 21 verbunden ist.

Umgekehrt kann bei einer logischen 0 des Taktsignals das erste Halbleiterschaltelement M1 geöffnet werden und das zweite Halbleiterschaltelement M2 geschlossen werden, so dass der Ausgangsanschluss der jeweiligen PWM-Stufe 23-i mit dem negativen Anschlusspunkt des Eingangsanschlusses 21 bzw. dem Bezugspotenzial verbunden ist. Bei einem Wechsel der Schaltzustände kann dabei zwischen dem Öffnen eines Halbleiterschaltelements M1, M2 und dem Schließen des komplementären Halbleiterschaltelements M2, M1 jeweils eine Totzeit vorgesehen sein, um einen möglichen Kurzschluss zu vermeiden. Es versteht sich jedoch, dass grundsätzlich neben der hier beschriebenen Schaltungskonfiguration einer PWM-Stufe 23-i grundsätzlich auch beliebige andere geeignete Schaltungsstrukturen möglich sein können.

Der Knotenpunkt K, welcher mit den Ausgangsanschluss der PWM-Stufen 23-i verbunden ist, ist, gegebenenfalls über einen Koppelkondensator 25, mit einem Anschlusspunkt der Primärseite eines Transformators 24 verbunden. Der zweite Anschlusspunkt der Primärseite des Transformators 24 kann mit einem Bezugspotenzial bzw. dem negativen Anschlusspunkt des Eingangsanschlusses 21 verbunden sein. Die sekundärseitigen Anschlusspunkte des Transformators 24 können mit dem Ausgangsanschluss 22 der Energieversorgungsvorrichtung 2 verbunden sein.

Es versteht sich, dass die hier beschriebene Schaltung der Energieversorgungsvorrichtung 2 grundsätzlich nur schematisch zur Veranschaulichung des Grundprinzips zur Erzeugung einer hochfrequenten Ausgangsspannung aus einer Eingangs-Gleichspannung zu verstehen ist. Je nach Anwendungsfall können darüber hinaus selbstverständlich auch beliebige weitere Komponenten, wie zum Beispiel Frequenzfilter o. ä. vorgesehen sein. Insbesondere können auch Spannungssensoren und/oder Stromsensoren, wie beispielsweise der in Figur 2 dargestellte Stromsensor 27 zur Erfassung der Eingangsstroms vorgesehen sein.

Für den Betrieb des elektrochirurgischen Instruments 1 sind mehrere Betriebsphasen möglich. Zum Beispiel kann während einer ersten Betriebsphase (Zündphase) zunächst ein Plasma 10 an der oder den Elektroden 11 des elektrochirurgischen Instruments 1 gezündet werden. Hierzu ist in der Regel eine relativ hohe elektrische Energie erforderlich. Darüber hinaus kann während einer zweiten Betriebsphase dieses Plasma 10 aufrecht erhalten werden. Für dieses Aufrechterhalten des Plasmas 10 ist eine geringere elektrische Energie erforderlich, als dies zum Zünden des Plasmas 10 der Fall ist. Andererseits ist zur Regelung dieses Plasmas 10 und der in diesem Plasma 10 umgesetzten elektrischen Energie eine möglichst präzise Regelung der elektrischen Leistung bzw. des elektrischen Stroms für das elektrochirurgische Instrument 1 erforderlich.

Um diesen komplementären Anforderungen für die erste Betriebsphase zum Zünden des Plasmas 10 sowie für die zweite Betriebsphase zum Aufrechterhalten des Plasmas 10 Rechnung zu tragen, kann die Steuereinrichtung 26 für die erste Betriebsphase und die zweite Betriebsphase eine unterschiedliche Ansteuerung der PWM-Stufen 23-i vorsehen.

Beispielsweise können in der ersten Betriebsphase für das Zünden des Plasmas 10 alle PWM-Stufen 23-i zeitgleich, d. h. mit einem gleichen PWM-Signal angesteuert werden. Bei einer solchen Ansteuerung mit einem gleichen PWM-Signal für alle PWM-Stufen 23-i ergibt sich somit an dem Knotenpunkt K ein rechteckförmiger Spannungsverlauf entsprechend dem PWM-Signal. Für eine Periodendauer einer vorgegebenen Frequenz der hochfrequenten Wechselspannung am Ausgang 22 der Energieversorgungsvorrichtung 2 erfolgt dabei in jeder PWM-Stufe 23-i jeweils ein Einschaltvorgang und jeweils ein Ausschaltvorgang.

In der zweiten Betriebsphase zum Aufrechterhalten des Plasmas 10 kann dagegen für jede PWM-Stufe 23-i ein individuelles, unterschiedliches PWM-Signal erzeugt werden. Insbesondere sind dabei für eine Periodendauer entsprechend der Frequenz des hochfrequenten Ausgangssignals der Energieversorgungsvorrichtung 2 auch mehrere Einschaltvorgänge und Ausschaltvorgänge möglich. Entsprechend erhöht sich die Anzahl der Schaltvorgänge in den PWM-Stufen 23-i. Durch diese gesteigerte Anzahl von Schaltvorgängen im Vergleich zu der ersten Betriebsphase erhöhen sich die Schaltverluste, wodurch die Effizienz in der Energieversorgungsvorrichtung 2 sinkt.

Andererseits kann durch diese Art der Ansteuerung die Signalform der hochfrequenten Ausgangsspannung besser gesteuert und insbesondere besser einem sinusförmigen Spannungsverlauf angepasst werden. Ferner kann durch diese Art der Ansteuerung auch der elektrische Strom bzw. die Ausgangsleistung der Energieversorgungsvorrichtung 2 genauer geregelt werden. Auf diese Weise kann auch die in dem Plasma 10 umgesetzte elektrische Energie und somit der Energieeintrag in ein Gewebe eines Patienten bei der Behandlung durch das elektrochirurgische Instrument 1 präzise geregelt werden. Da während dieser zweiten Betriebsphase zum Aufrechterhalten des Plasmas 10 die erforderliche elektrische Leistung geringer ist als während der ersten Betriebsphase zum Zünden des Plasmas 10, ist die damit verbundene geringere Effizienz aufgrund der erhöhten Anzahl von Schaltvorgängen von untergeordneter Bedeutung.

Zur Ansteuerung der einzelnen PWM-Stufen 23-i während der zweiten Betriebsphase zum Aufrechterhalten des Plasmas 10 kann die Steuereinrichtung 26 die PWM-Signale für die einzelnen PWM-Stufen 23-i beispielsweise auf Grundlage des Konzepts einer sogenannten Multiphasen-PWM-Regelung ausführen. Da das grundlegende Konzept einer solchen Multiphasen-PWM-Regelung jedoch einer konventionellen Multiphasen-PWM Steuerung entsprechen kann, erübrigt sich hier eine detailliertere Ausführung.

Die erste Betriebsphase zum Zünden des Plasmas 10 kann beispielsweise für eine vorbestimmte Zeitspanne von beispielsweise 200 ms, 500 ms oder eine beliebige andere geeignete Zeitspanne ausgeführt werden. Anschließend kann gegebenenfalls automatisch in die zweite Betriebsphase zum Aufrechterhalten des Plasmas 10 übergegangen werden. Ferner ist es auch möglich, beispielsweise während der ersten Betriebsphase, die elektrische Leistung, beispielsweise durch Überwachung eines elektrischen Stroms, zum Beispiel durch Überwachung des Eingangsstrom mittels eines Stromsensors 27 zu ermitteln und hieraus auf ein erfolgreiches Zünden des Plasmas 10 zu schließen. Da nach dem Zünden des Plasmas 10 der elektrische Strom signifikant ansteigen wird, kann aus der Überwachung des Stromverlaufs auf den Zeitpunkt zum Zünden des Plasmas 10 geschlossen werden. Somit kann nach der Detektion des erfolgreichen Zündens des Plasmas 10 in die zweite Betriebsphase übergegangen werden.

Während des Aufrechterhaltens des Plasmas 10 in der zweiten Betriebsphase können beliebige geeignete Betriebsparameter, wie beispielsweise ein elektrischer Strom, wie zum Beispiel ein Eingangsstrom, auf Grundlage der (Ausgabe-)Sensorwerte des Stromsensors 27 erfasst und überwacht werden. Auf diese Weise kann die in dem Plasma 10 umgesetzte elektrische Energie geregelt und insbesondere begrenzt werden.

Ferner ist es auch beispielsweise durch Überwachung von Betriebsparametern, wie zum Beispiel des Eingangsstroms, möglich, ein Erlöschen des Plasmas 10 zu erkennen. Wird ein solches Erlöschen des Plasmas 10 detektiert, so kann gegebenenfalls erneut in die erste Betriebsphase zum Zünden des Plasmas 10 übergegangen werden. Zusätzlich oder alternativ ist jedoch auch beispielsweise ein manuelles Triggern zum Zünden des Plasmas 10 durch die erste Betriebsphase möglich.

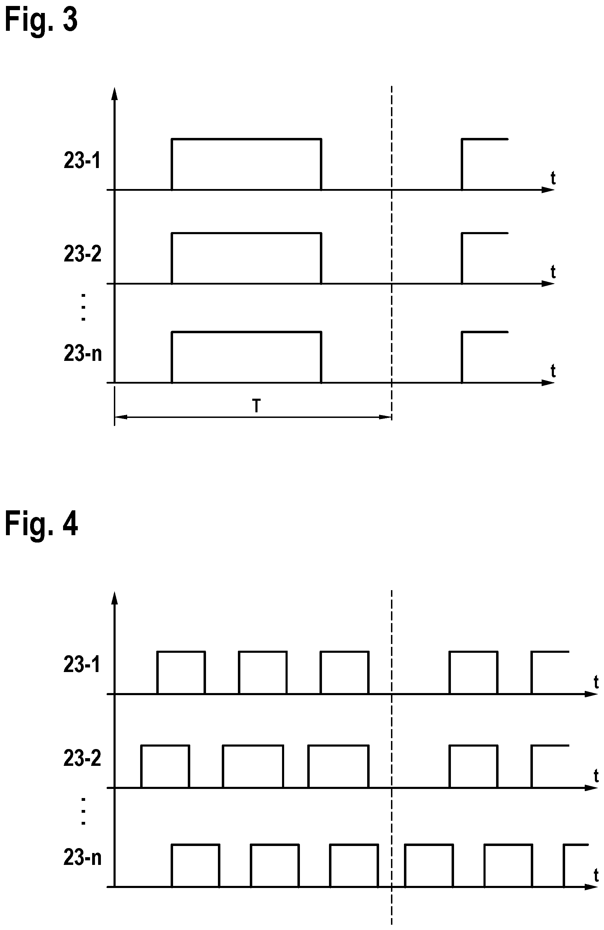

Figur 3 zeigt eine schematische Darstellung von Timing-Diagrammen für Ansteuersignale der PWM-Stufen 23-i in einer ersten Betriebsphase. Wie hierbei zu erkennen ist, werden die einzelnen PWM-Stufen 23-i jeweils mit einem gleichen Ansteuersignal, d. h. mit Ansteuersignalen, welche zu identischen Zeitpunkten schalten, beaufschlagt.

Figur 4 zeigt eine schematische Darstellung von Timing-Diagrammen für die Ansteuerung von PWM-Stufen 23-i in einer zweiten Betriebsphase. Wie hierbei zu erkennen ist, werden die einzelnen PWM-Stufen 23-i jeweils mit individuellen Ansteuersignalen beaufschlagt. Die Schaltvorgänge in den einzelnen PWM-Stufen 23-i können dabei zu unterschiedlichen Zeitpunkten erfolgen. Darüber hinaus können auch für eine Periodendauer T der hochfrequenten Ausgangssignale mehrere Ein- und Ausschaltvorgänge je PWM-Stufe 23-i vorgesehen sein.

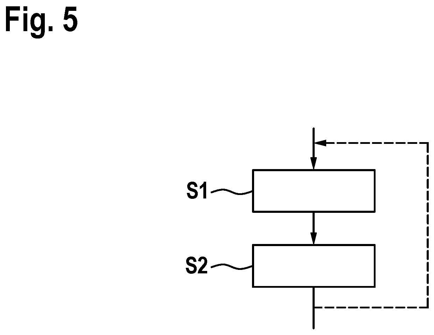

Figur 5 zeigt ein Ablaufdiagramm, wie es einem Verfahren zum Bereitstellen einer hochfrequenten Versorgungsspannung für ein elektrochirurgisches Instrument gemäß einer Ausführungsform zugrunde liegen kann. Das Verfahren kann beispielsweise in einer zuvor beschriebenen Energieversorgungsvorrichtung 2 implementiert werden. Somit gelten sämtliche zuvor in Zusammenhang mit der Energieversorgungsvorrichtung 2 gemachten Ausführungen auch für das nachfolgend beschriebene Verfahren. Umgekehrt kann die Energieversorgungsvorrichtung 2 auch in beliebiger geeigneter Weise ausgeführt sein, um das nachfolgend beschriebenen Verfahren zu realisieren.

In einem Schritt S1 erfolgt zunächst ein zeitsynchrones Ansteuern mehrerer PWM-Stufen 23-i. Diese erste Betriebsphase dient, wie zuvor bereits ausgeführt, dem Zünden eines Plasmas 10 an dem elektrochirurgischen Instrument 1.

Nach dieser ersten Betriebsphase können in einem Schritt S2 die mehreren PWM-Stufen in einer zweiten Betriebsphase betrieben werden, in welcher die mehreren PWM-Stufen zeitversetzt angesteuert werden. Diese zweite Betriebsphase dient, wie ebenfalls zuvor ausgeführt, dem Aufrechterhalten des Plasmas 10. Insbesondere können in dieser zweiten Betriebsphase für eine Periodendauer T eines auszugebenden hochfrequenten Ausgangssignals mehrere Ein- und Ausschaltvorgänge vorgesehen sein.

Erlischt das Plasma 10 während des Betriebs, so kann gegebenenfalls erneut, insbesondere automatisch, zu der ersten Betriebsphase gewechselt werden.

Zusammenfassend betrifft die vorliegende Erfindung die Energieversorgung eines elektrochirurgischen Instruments. Hierbei sind mehrere Betriebsphasen vorgesehen. In einer ersten Betriebsphase zum Zünden eines Plasmas an dem elektrochirurgische Instrument werden mehrere PWM-Stufen zeitsynchron angesteuert, um einen rechteckförmigen Spannungsverlauf zu generieren. In einer zweiten Betriebsphase zum Aufrechterhalten des Plasmas werden die mehreren PWM-Stufen individuell und mit gegebenenfalls mehreren Schaltvorgängen pro Periodendauer des zu generierenden Ausgangssignals angesteuert.

Die Zeichnungen, die Beschreibung und die Ansprüche enthalten zahlreiche Merkmale in Kombination. Es versteht sich, dass die vorstehend genannten Merkmale nicht nur in der jeweils angegebenen Kombination, sondern auch in anderen Kombinationen oder in Alleinstellung verwendbar sind, ohne den Rahmen der vorliegenden Erfindung zu verlassen. Energieversorgungsvorrichtung für ein elektrochirurgischen Instruments. Hierbei sind mehrere Betriebsphasen vorgesehen. In einer ersten Betriebsphase zum Zünden eines Plasmas an dem elektrochirurgischen Instrument werden mehrere PWM-Stufen zeitsynchron angesteuert, um einen rechteckförmigen Spannungsverlauf zu generieren. In einer zweiten Betriebsphase zum Aufrechterhalten des Plasmas werden die mehreren PWM-Stufen individuell und mit gegebenenfalls mehreren Schaltvorgängen pro Periodendauer des zu generierenden Ausgangssignals angesteuert.

## Patentansprüche

1. Energieversorgungsvorrichtung (2) für ein elektrochirurgisches Instrument (1), mit:
mehreren PWM-Stufen (23-i), mit jeweils einem Eingangsanschluss und einem Ausgangsanschluss, wobei jede PWM-Stufe (23-i) dazu ausgelegt ist, an ihrem Eingangsanschluss mit einer Gleichspannungsquelle (3) elektrisch gekoppelt zu werden, und wobei die Ausgangsanschlüsse der mehreren PWM-Stufen an einem gemeinsamen Knotenpunkt (K) elektrisch miteinander gekoppelt sind;
einem Transformator (24), mit einer Primärseite und einer Sekundärseite, wobei ein erster Anschlusspunkt der Primärseite des Transformators (24) mit dem gemeinsamen Knotenpunkt (K) der mehreren PWM-Stufen (23-i) elektrisch gekoppelt ist, und
wobei der Transformator (24) dazu ausgelegt ist, zwischen einem ersten Anschlusspunkt und einem zweiten Anschlusspunkt der Sekundärseite eine Versorgungsspannung für das elektrochirurgische Instrument (1) bereitzustellen; und
einer Steuereinrichtung (26), die dazu ausgelegt ist, Ansteuersignale für die mehreren PWM-Stufen (23-i) bereitzustellen, wobei die Steuereinrichtung (26) dazu ausgelegt ist, in einem ersten Betriebsmodus die mehreren PWM-Stufen (23-i) zeitsynchron anzusteuern, und in einem zweiten Betriebsmodus die mehreren PWM-Stufen (23-i) zu unterschiedlichen Zeitpunkten anzusteuern.

2. Energieversorgungsvorrichtung (2) nach Anspruch 1, wobei die Steuereinrichtung (26) dazu ausgelegt ist, die mehreren PWM-Stufen (23-i) in dem zweiten Betriebsmodus mit einer Taktrate anzusteuern, welche größer ist als die Taktrate in dem ersten Betriebsmodus.

3. Energieversorgungsvorrichtung (2) nach Anspruch 1 oder 2, wobei die Ansteuerung in dem zweiten Betriebsmodus eine Multiphasen-PWM-Ansteuerung umfasst.

4. Energieversorgungsvorrichtung (2) nach einem der Ansprüche 1 bis 3, wobei eine Amplitude der Versorgungsspannung für das elektrochirurgische Instrument (1) zwischen dem ersten Anschlusspunkt und einem zweiten Anschlusspunkt der Sekundärseite des Transformators (24) größer ist als eine elektrische Eingangsspannung der Gleichspannungsquelle (3), die mit den Eingangsanschlüssen der PWM-Stufen (23-i) elektrisch gekoppelt ist.

5. Energieversorgungsvorrichtung (2) nach einem der Ansprüche 1 bis 4, wobei die Eingangsspannung der Gleichspannungsquelle (3) kleiner oder gleich 60 Volt ist.

6. Energieversorgungsvorrichtung (2) nach einem der Ansprüche 1 bis 5, mit einem Koppelkondensator (25), der zwischen dem gemeinsamen Knotenpunkt (K) der mehreren PWM-Stufen (23-i) und dem ersten Anschlusspunkt der Primärseite des Transformators (24) angeordnet ist.

7. Energieversorgungsvorrichtung (2) nach einem der Ansprüche 1 bis 6, wobei die Steuereinrichtung (26) dazu ausgelegt ist, einen elektrischen Eingangsstrom von der Gleichspannungsquelle (3) zu den Eingangsanschlüssen der PWM-Stufen (23-i) zu ermitteln, und die mehreren PWM-Stufen (23-i) unter Verwendung des ermittelten Eingangsstroms anzusteuern.

8. Energieversorgungsvorrichtung (2) nach einem der Ansprüche 1 bis 7, wobei die Steuereinrichtung (26) dazu ausgelegt ist, die mehreren PWM-Stufen (23-i) in dem ersten Betriebsmodus anzusteuern, falls an einem an dem Ausgangsanschluss angeschlossenen elektrochirurgischen Instrument (1) kein Plasma (10) detektiert wird.

9. Energieversorgungsvorrichtung (2) nach einem der Ansprüche 1 bis 8, wobei die Steuereinrichtung (26) einen FPGA umfasst, der dazu ausgelegt ist, die Ansteuersignale für die mehreren PWM-Stufen (23-i) zu erzeugen und bereitzustellen.

10. Energieversorgungsvorrichtung (2) nach einem der Ansprüche 1 bis 9, wobei die Steuereinrichtung (26) dazu ausgelegt ist, nach einer vorbestimmten Zeitspanne von dem ersten Betriebsmodus in den zweiten Betriebsmodus zu wechseln.

11. Energieversorgungsvorrichtung (2) nach einem der Ansprüche 1 bis 10, wobei die mehreren PWM-Stufen (Trend 20-i) jeweils eine Halbbrücke mit zwei Halbleiterschaltelementen umfassen.

12. Energieversorgungsvorrichtung (2) nach einem der Ansprüche 1 bis 11, wobei die Steuereinrichtung (26) dazu ausgelegt ist, die mehreren PWM-Stufen (23-i) jeweils mit einer Taktfrequenz von mindestens 300 kHz anzusteuern.

13. Elektrochirurgisches System, mit:
einem elektrochirurgischen Instrument (1) mit mindestens einer Elektrode (11) zur Erzeugung eines Plasmas (10); und
einer Energieversorgungsvorrichtung (2) nach einem der Ansprüche 1 bis 12.

14. Elektrochirurgisches System nach Anspruch 13, mit einer Gleichspannungsquelle (3), die dazu ausgelegt ist, an den Eingangsanschlüssen der mehreren PWM-Stufen (23-i) eine Gleichspannung mit einer vorbestimmten Spannungshöhe bereitzustellen.

15. Verfahren zum Bereitstellen einer hochfrequenten Versorgungsspannung für ein elektrochirurgisches Instrument (1) mit einer Energieversorgungsvorrichtung (2) nach einem der Ansprüche 1 bis 12, mit den Schritten:
zeitsynchrones Ansteuern (S 1) der mehreren PWM-Stufen (23-i) in einer ersten Betriebsphase zum Zünden eines Plasmas (10) an dem elektrochirurgischen Instrument (1), und
zeitlich versetztes Ansteuern (S 2) der mehreren PWM-Stufen (23-i) in einer zweiten Betriebsphase zum Aufrechterhalten des Plasmas (10) an dem elektrochirurgischen Instrument (1).
